Europäisches Patentamt

European Patent Office

Office européen des brevets

(11) Publication number: 0 348 143
A2

# EUROPEAN PATENT APPLICATION

(21) Application number: 89306208.3

(51) Int. Cl.⁴: C07H 11/00 , A61K 31/70

(22) Date of filing: 20.06.89

| | |
|---|---|
| The title of the invention has been amended (Guidelines for Examination in the EPO, A-III, 7.3). (30) Priority: 21.06.88 US 209372<br>20.09.88 US 246755<br><br>(43) Date of publication of application:<br>27.12.89 Bulletin 89/52<br><br>(84) Designated Contracting States:<br>AT BE CH DE ES FR GB GR IT LI LU NL SE | (71) Applicant: MARION LABORATORIES, INC. (a Delaware corporation)<br>9300 Ward Parkway<br>Kansas City Missouri 64114(US)<br><br>(72) Inventor: Bowen, S. Marc<br>706 Westwood Drive<br>Independence Missouri 64050(US)<br>Inventor: Bodine, Richard S.<br>4340 Harrison<br>Kansas City Missouri 64110(US)<br>Inventor: Coleman, James C.<br>9948 Westgate Lane<br>Lenexa Kansas 66215(US)<br>Inventor: Cole, Douglas L.<br>15625 Overbrook Lane<br>Stanley Kansas 66224(US)<br><br>(74) Representative: Selby-Lowndes, Guy Francis Charles<br>Moonrakers Durfold Wood<br>Plaistow Billinghurst West Sussex RH14 0PL(GB) |

(54) Bismuth derivatives of saccharide phosphates and sulphates.

(57) The titled compositions are useful as pharmaceuticals in ameliorating disorders associated with gastric mucosal damage. For example, compositions containing a complex salt of bismuth hydroxide sucrose octasulfate can be thus employed. Bismuth (phosph/sulf)ated saccharides are also useful in ameliorating gastrointestinal disorders associated with Campylobacter-like organisms such as, for example, Campylobacter pylori. For example, compositions containing the complex salt of bismuth hydroxide sucrose octasulfate can also be thus employed to treat Type B gastritis.

# BISMUTH (PHOSPH/SULF)ATED SACCHARIDES

## Field

This invention concerns bismuth phosphorylated and/or sulfonated saccharides, with procedures for preparation and use thereof. These saccharides in general are useful as pharmaceuticals.

## Background

Sucralfate is a well-known anti-ulcerative. See e.g., Windholz et al. (Eds.), The Merck Index, 10th Ed., Merck & Co., Inc., Rahway, N.J. (1983), at page 1273, entry 8755. See ref., Nitta et al., U.S. Pat. No. 3,432,489 (Mar. 11, 1968), which describes a disaccharide polysulfate aluminum compound and method.

Berkow et al. (Eds.), The Merck Manual, 15th Ed., Merck Sharpe & Dohme Research Laboratories Div. of Merck & Co., Inc., Rahway, N.J. (1987), at page 744 reports in regard to treatment of pepticulcer that sucralfate is beneficial in treatment of all such ulcers, and several bismuth-containing preparations (including colloidal bismuth subcitrate) are effective in such treatment with an action similar to sucralfate.

PEPTO BISMOL is one well-known digestion aid having bismuth subsalicylate therein. See further, Barnhart et al. (Eds.), Physicians' Desk Reference, 41st Ed., Medical Economics Co., Inc., Oradell, N.J. (1987), at page 1590.

Feldman, Am. J. Med. Sci., 288 (3), 136-48 (1984), at page 144 reports that the mechanism of action of bismuth subsalicylate is uncertain and that bismuth subcarbonate does not behave similarly thereto. This may suggest compound-specific action for bismuth-containing compositions.

The Merck Index, 10th Ed., ibid., at pages 177-80 reports on bismuth and some of its compounds. Bismuth, for example, is reported to have been used in veterinary medicine externally in dusting powders for indolent, moist or suppurating lesions, internally as a protecterant of the gastrointestinal lining and has been recommended to treat buccal (cheek) warts in dogs.

Michaeli, Eur. Pat. Appl. Pub. No. 0 230 023 published on July 29, 1987, publishes on pharmaceutical compositions for the enhancement of wound healing. The compositions comprise sulfated saccharides, particularly mono- and disaccharides, or their salts, to enhance healing of wounds. Preferred salts are the soluble salts, most preferably the alkali metal salts, most particulrly potassium and sodium salts. Sucrose octasulfate is the most preferred sulfated saccahardie. The saccharides may be present in any form, including liquids, gels or time-release polymers. In preferred practice, the saccharide is present in combination with with collagen.

Kuperus, U.S. Pat. No. 4,581,221 (Apr. 8, 1986), describes ulcer detection, with Tc-99m. Suitable ulcer-specific compounds for use therein include the metal or basic salts of maximally sulfated sucrose, Lower molecular weight compounds are preferred for solubility and a quick gastrointestinal tract passage to leave the Tc-99m for the detection.

Kooperman et al., U.S. Pat. No. 3.379,717 (Apr. 23, 1968), describes bismuth subglycerrhizinate and method of preparing same. The compound agrees with the formula C41 H61 014 COOBi and accelerates the healing of gastric ulcers.

Marshall, Eur. Pat. Appl. No 0 206 627 published on December 30, 1986, publishes methods for the treatment of gastrointestinal disorders. The methods of that publication comprise treatment of humans or lower animals, having an infectious gastrointestinal disorder, by administering bismuth, preferably as a pharmaceutically acceptable salt. The disorder may be caused or mediated by Campylobacter-like organisms, e.g., Campylobacter pylori.

Borody, PCT Int. Publ. No. WO 86/05981 published on Octoer 23, 1986, publishes a treatment of non-ulcer dyspepsia with bismuth salts. The dyspepsia is associated with Campylobacter pylori infection, and the administration of the bismuth salts in association with antibiotics was reported to be preferred and particularly efficacious.

Graham et al., The Americn Journal of Gastroenterology, 82, 283-6 (1987), reports on Campylobacter pylori gastritis: The past, the present, and specultion about the future. See also, Blaser, Gastroenterology, 93, 371-83 (1987).

Scrip (December 3, 1986) reports on bacteria and the anti-ulcer market, which speculates on the possibility of a spurt in the growth of bismuth products due to the theory that Campylobacter pylori infection is associated with ulcer development and which also specifically reports on use of DE-NOL plus an antibiotic. But see, Borsch, Leber, Magen, Darm. (W. Ger.), 18, 38-45 (January, 1988), which reports on therapy for Campylobacter pylori infection. Borsch reports that a simple and efficient therapy for eradicating Campylobacter pylori infection with high reliability from gastroduodenal mucosae is unknown, and that bismuth salts might play an important future role in a more pragmatic approach which investigates any potential clinical benefit derived from a suppression of bacterial growth, possibly resulting in a temporary restoration of the mucosal integrity, but that this approach will probably fail to modify the long-term natural history of Campylobacter pylori-associated chronic gastritis and its sequelae.

## Summary

The present invention, in one aspect, provides bismuth (phosph/sulf)ated saccharides. Another aspect provides a process for preparing a bismuth (phosph/sulf)ated saccharide comprising contacting a hydrogen (phosph/sulf)ated saccharide and a bismuth substance under condition such that the bismuth (phosph/sulf)-ated saccharide is prepared. A further aspect provides a method for ameliorating disorders associated with gastric mucosal damage of a subject comprising administering a bismuth (phosph/sulf)ated saccharide to the subject under conditions such that the gastric mucosal damage is treated. The present invention also provides a method for ameliorating a gastrointestinal disorder of a subject associated with a Campylobacter-like organism (phosph/sulf)ated saccharide so the subject under conditions such that said disorder is ameliorated.

The bismuth (phosph/sulf)ated saccharides are useful as pharmaceuticals. For instance, they are especially useful, in general, in the amelioration of such disorders as noted herein that are associated with gastric mucosal damage. An notably, the lattermost characteristic method summarized above not only can most significantly reduce populations of the Campylobacter-like organism, but also, it can provide a most significantly effective decrease in inflammation of treated gastrointestinal mucosa. Further significant advantages inherently attend this invention as well.

## Illustrative Detail

In general, the bismuth (phosph/sulf)ated saccharides are compositions which contain such a component as a phosphorylated and/or a sulfonated saccharide, which is a sacchride generally having more than one moiety selected from such moieties as at least one of phosphate and sulfate moieties esterified thereto.

The component such as bismuth includes such a metallic element as bismuth and pharmaceutically acceptable compounds therewith. The pharmaceutically acceptable compounds with bismuth include, of course, molecular level covalent or ionic complexes with bismuth and the phosphated and/or sulfated saccharide moieties, molecular level covalent or ionic complexes with such bismuth-containing moieties or compounds as bismuth hydroxides and the phosphated and/or sulfated saccharide moieties, these compositions in the presence of a suitable pharmaceutical carrier.

The component such as a sulfate ester and/or a phosphate ester saccharide includes thus such saccharides as 1) sulfated saccharides, 2) phosphated saccharides, 3) sulfated-phosphated saccharides, and 4) mixtures thereof. Saccharide components having at least three sulfate ester moieties per saccharide nucleus are desirably employed. Polysulfated saccharides are more typically employed in the practice of this invention. The polysulfated saccharides desirably contain substantial amounts of persulfated saccharides.

Saccharide moieties themselves which may be employed in the practice of this invention include mono-, di-, tri-, tetra-, and oligosaccharides. Examples of suitable saccharide nuclei or moieties may be selected from appropriate residues of such saccharides as erythrose, threose, arabinose, deoxyribose, fructose, glucose, ribose, mannose, lactose, cellobiose, maltose, sucrose, trehalose, melezitose and stachyose. The saccharide moieties desirably are disaccharides of pentoses and/or hexoses. Sucrose is preferred.

The bismuth (phosph/sulf)ated saccharides may be a composite mixture, i.e., a composition combining more than one chemical entity to make up the composition. They may be considered complex bismuth salts of (phosph/sulf)ated saccharide(s).

The bismuth saccharides of this invention are generally insoluble in water, lower alcohols, e.g., methanol, lower ketones, e.g., acetone, dilute aqueous hydrochloric acid, e.g., 0.1 N, with generally no gelling propensity in acidic water. They may be semi-crystalline in the solid state.

Significantly, the bismuth (phosph/sulf)ated saccherides employed in the practice of this invention are generally and quite surprisingly soluble in appropriate aqueous ammonia solutions. This is most notably significant because the vicinity surrounding such Campylobacter-like organisms as, for example, Campylobacter pylori is typically ammoniacal, particularly in vivo. See e.g., Graham et al., supra., at page 284, and Blaser, supra., at page 378.

Herein, a procedure is a method and/or process.

In general, the bismuth (phosph/sulf)ated saccharide can be prepared by contacting a hydrogen (phosph/sulf)ated saccharide and a bismuth substance. Conditions are those sufficient to form the bismuth saccharides of this invention.

In general, the hydrogen (phosph/sulf)ated saccharide is a saccharide analogous to that of the corresponding bismuth (phosph/sulf)ated saccharide but having phosphoric and/or sulfonic acid moieties bonded therewith. Sulfonic acids are preferred.

The hydrogen (phosph/sulf)ated saccharides can be obtained, or can be prepared by known procedures or by procedures analogous thereto. For example, the following hydrogen (phosph/sulf)ated saccharides are in general commercially available:

2$'$-deoxy-a-D-ribose-1-phosphate;
2$'$-deoxyribose-5$'$-phosphate;
D-fructose-1-phosphate;
D-fructose-6-phosphate;
D-fructose-1,6-diphosphate;
alpha-D-galactose-1-phosphate;
D-galactose-6-phosphate;
galactose-6-sulfate;
glucose-1-phosphate;
D-glucose-1-phosphate;
glucose-6-phosphate;
D-glucose-6-phosphate;
glucose-1,6-diphosphate;
D-glucose-6-sulfate (sodium salt);
alpha-mannose-6-phosphate;
alpha-lactose-phosphate (barium salt).

See e.g., Anderson et al. (Eds.), Chem Sources U.S.A., Directories Publishing Co., Inc., Ormond Beach, Fla. (1984). Also, phosphorylation and/or sulfonation may be accomplished on corresponding saccharides having appropriate esterification site(s) available, as is known in the art. For example, phosphorylation may be accomplished by appropriate treatment of the reactant saccharide with a suitable phosphorylating agent, e.g., one which may be phosphoryl chloride or cyanoethyl phosphate. See e.g., Carey et al., Advanced Organic Chemistry, Part B: Reactions and Synthesis, Plenum Press, New York (1977), at pages 482-96 & 507. Sulfonation may be accomplished by appropriate treatment of the reactant saccharide with a suitable sulfating agent, e.g., chlorosulfonic acid, anhydrous sulfuric acid or sulfurtrixode-pyridine complex in a solvent such as pyridine, formamide, dimethyl formamide, chloroform or liquid sulfur dioxide. See e.g., Nitta et al., US 3432489. See also, Michaeli, EP 0230023, especially for preparations with sodium and sulfate saccharides.

Metal salts of the (phosph/sulf)ated saccharides may be converted to the hydrogen (phosph/sulf)ated saccharides by known procedures. For example, ion exchange procedures with an ion exchange resin such as a sulfonated divinyl benzene resin in its hydrogen ion form may be thus employed, passing the metal salts, desirably the potassium salts, over the resin in a suitable media, e.g., water, especially in a highly pure water in an about 20:1 weight-weight ratio of the water to metal salt, at room temperature in order to prepare the hydrogen (phosph/sulf)ated saccharides. The thus-prepared hydrogen (phosph/sulf)ated saccharides may be cooled to 10 degrees C. or so or kept at temperatures from 20 to 30 degrees C. at this stage, if desired.

In general, the bismuth substance contains bismuth, which, when contacted with the hydrogen (phosph/sulf)ated saccharides, can form the bismuth (phosph/sulf)ated saccharide. As an illustration, the bismuth substance may be bismuth hydroxide (Bi(OH)3). Freshly prepared Bi(OH)3 is preferred. Commercially available Bi(OH)3 may contain Bi2O3 and may not work as well in the practice of this invention.

Typically, the hydrogen (phosph/sulf)ated saccharide is dispersed or dissolved in a suitable medium, e.g., water for the contact. Concentrations of the hydrogen (phosph/sulf)ated saccharide may vary widely, but higher aqueous concentrations, at accordingly low pH values, e.g., pH 1.5, may advantageously be about room temperature, e.g., 25 degrees C. The bismuth substance is contacted with the hydrogen saccharide dispersion or solution. Typically, the bismuth substance is a solid, and it may advantageously be stirred into the dispersion or solution of the hydrogen (phosph/sulf)ated sacchride, typically in water. Alternatively, for example, the bismuth substance may be slurried in water as a sample of fine particles,

4

with the typically aqueous dispersion or solution of the hydrogen (phosph/sulf)ated saccharide being added thereto, e.g., slowly as from immediate preparation from the mentioned ion exchange procedure using an ion exchange column. Amounts of the hydrogen (phosph/sulf)ated saccharide may advantageously be in a stoichiometric equivalent excess in comparison to the bismuth substance, e.g., from above 100 to 160 percent of theory or more in such an excess. Thus accordingly, e.g., the bismuth substance may be used in a molar ratio of from 5:1 to 8:1 in comparison to the hydrogen (phosph/sulf)ated saccharide. Cooling during this contact step may be undertaken if desired, but it is not generally necessary if the contact is carried out at room temperature. This mixture may be allowed to be stirred for a time, say, from a score (20) minutes to an hour or so. e.g., half an hour. The bismuth saccharide product is typically recovered as a solid.

The recovery of the product may advantageously be carried out by suction filtration and washing, with vacuum drying. The washing may generally be with such liquids as, e.g., water, methanol and/or acetone, The product may be slurried in methanol and/or acetone and suction filtered therefrom if desired. Heating of the product to dry it should be avoided because temperatures above usual ambient temperatures, e.g., 45 degrees C., may cause decomposition of the desired product. The product is thus advantageously stored under refrigeration, e.g., in a freezer, generally with cautionary protection from exposure to actinic radiation as may be provided with an amber or opaque glass bottle.

Yields of the product can be high. The yields can be as high as at least about 90 percent of theory or even about 95 percent of theory or more.

Procedures for use of the bismuth (phosph/sulf)ated saccharide generally involve treating gastric mucosal damage of a subject by orally administering an amount of the bismuth (phosph/sulf)ated saccharide effective to treat the damage. For instance, gastric mucosal damage may be the result of exposure to ethanol, excess acid and/or excess bile salts. Treatment of the gastric mucosal damage with the compositions of this invention ameliorates, or even eliminates, the damage.

Suitable amounts of the bismuth (phosph/sulf)ated saccharide generally include those which may be in doses from 5 to 100 mg per kg body weight of subject par dose, say, 20 mg per kg per dose. Multiple doses per day are typical, often, say four doses per day.

The method for use of the bismuth (phosph/sulf)ated saccharide in the practice of this invention can also generally involve treating a gastrointestinal disorder of a subject associated with a Campylobacter-like organism by administering an amount of the bismuth (phosph/sulf)ated saccharide effective to ameliorate the disorder. Preferably, the effect is substantial or is even great.

The administration is preferably oral.

The subject can be a human patient.

The gastrointestinal disorder typically includes or is so-called Type B gastritis,. See e.g., Graham et al., supra., at page 283,.

Suitable amounts of the bismuth (phosph/sulf)ated saccharide here also include those which may be in doses about from 5 to 100 mg per kg of body weight of the subject per dose, also say, about 20 mg per kg per dose. Also here in human patients, an estimated range of 0.5 to 10 grams per day is contemplated. Divided doses per day are typical, often, say, four oral doses per day, especially with human patients.

In general, the Campylobacter-like organism can be any such organism which is the object which appropriately responds to the method of this invention. Campylobacter-like organisms may include those known as such in the art. See e.g., Marshall, EP 0206627, at page 4 & Blaser, supra. A Campylobacter pylori organism population may contain a notable population of Campylobacter pylori. Typically, Campylobacter pylori and its associated gastrointestinal disorders, e.g., the Type B gastritis, are advantageously treated effectively.

Dosage forms of the bismuth (phosph/sulf)ated saccharide may include such unit dosage forms as tablets, capsules and appropriate suspensions. Tablet forms may be advantageously employed because of ease of manufacture and administration.

Accordingly, numerous advantages attend this invention.

The following examples further illustrate the invention. Percentages are by weight unlss otherwise specified.

Example 1

To a 12-L 3 necked flask fitted with a mechanical stirrer and separatory/addition funnel was charged 1750 mL of glacial acetic acid and 3500 mL of "nanopure" water as obtained by passing deionized water through a NANOpure II (barnstead) column. To the acid mixture was added 500 g of Bi(NO2)3 5H2O. The mixture was stirred to dissolve the solid. Next, with cooling, 3500 mL of concentrated (28%) NH4OH (aq.)

was slowly added. The pH of the mixture became alkaline. Solid product was collected by filtration. The filtered solid was washed with "nanopure" water and next with methanol. The filtered solid was dried under vacuum at 50 degrees C., which yielded 253.2 g of product Bi(OH)3 (94.6 percent of theory).

A sample of 1600 g of amberlite IR-120 ion exchane resin which had been washed with 2-3 bed volumes of 1 N HCl (aq.) was loaded onto a column. The loaded resin was washed with "nanopure" water until the pH became nearly that of the water and until no chloride precipitate was observed with argentous nitrate.

A sample of 80 g of potassium sucrose octasulfate (K8SOS), which was previously prepared according to Examples 1 & 2 of Michaeli, EP 0230023, was dissolved in about 1400 mL of "nanopure" water. This K8SOS solution was loaded onto the resin of the column and was eluted fairly rapidly with 4 L of "nanopure" water.

Effluent from the column was directed to a 12-L 3-necked flask containing a slurry of the above Bi(OH)3 in 1 L of "nanopure" water. The slurry was cooled by an ice-water bath. The latter reaction mixture was next stirred for 30 minutes; whereupon it was filtered slowly with suction with several Buchner funnels. Suction was maintained overnight, and upon drying the product was ground to a finely divided state. The yield of product was 139.42 g (95.5 percent of theory). The product was odorless, white, semi-crystalline as determined by x-ray powder diffraction procedures, was insoluble in water, 0.1 N HCl (aq.) methanol and acetone, was soluble in 4 N sulfuric acid (aq.) and soluble in trifluoroacetic acid but decomposed gradually in these latter two acid media, and had the following composition:

Octasulfate content: 88.6 percent (HPLC & EA);

Heptasulfate content: 5.4 percent (HPLC & EA);

Hydroxide content as, e.g., Bi(OH)3: 5.9 percent (HPLC & EA);

Bismuth content: 58.6 percent (AA & EA);

Water content: ca. 7.8 percent (Difference & Karl Fisher);

Microorganisms: None detected (Microbial bioburden).

The molecular structure was confirmed by Fourier transform infrared spectroscopy (FT-IR), by high pressure liquid chromatograph (HPLC) against a known reference standard of K8SOS-7H2O and by atomic absorption (AA) spectroscopy. Elemental analyses (EA) were employed in the above. Thus, the product may be considered to be a complex salt of bismuth hydroxide sucrose octasulfate or substantially empirically [Bi (OH)2] 8SOS.


Example 2

The effectiveness of the [Bi (OH)2] 8SOS as from Example 1 as an antiulcer agent was determined by examining its actions in three animal models of gastric mucosal damage. Oral administration of 95% ethanol caused extensive gastric mucosal damage and the [Bi (OH)2] 8SOS was effective in reducing the severity of damge in this ethanol-induced gastric mucosal damage model. This model serves as a screen for antiulcer activity. Oral administration of 0.6 M hydrochloric acid caused extensive gastric mucosal damage and the [Bi (OH)2] 8SOS was effective in reducing the severity in this hydrochloric acid-induced gastric mucosal damage model. This model also serves as a screen for antiulcer activity. When the pylorus is ligated (Shay rat model), gastric secretions accumulating over a time span of 14 hours caused extensive damage to the forestomach of the rat. The [Bi (OH)2] 8SOS was effective in reducing the severity of lesions induced in the Shay rat model. This model also serves as a screen for antiulcer activity.

Male Sprague-Dawley rats weighing approximately 200 g were used in these studies. They were maintained in individual hanging stainless steel cages, fasted for 48 hours prior to the start of the study and allowed free access to drinking water until the study commenced. The concentration of the [Bi (OH)2] 8SOS used in each study was 122.9 mg/mL IUPAC. The [Bi (OH)2] 8SOS was insoluble in deionized water and sonication was required for dosing. The [Bi (OH)2] 8SOS was administered orally in a volume of 1 mL via an oral dosing needle.

Determination of protective characteristics of the (Bi (OH)2] 8SOS against ethanol-induced gastricmucosal damage was undertaken. Rats were given 1 mL of the [Bi (OH)2] 8SOS orally fifteen minutes prior to oral administration of 1 mL of 95% ethanol. One hour later, stomachs were removed, and the mucosal surface was examined macroscopically for damage. The degree of damage was graded according to the scale presented in Table 1 below.

6

TABLE 1

| GRADE OF OBSERVED MUCOSAL DAMAGE | |
|---|---|
| Grade | Appearance of Gastric Mucosa |
| 0 | Normal mucosa |
| 1 | Slight edema and congestion |
| 2 | Edema, congestion and bleeding |
| 3 | 1 or 2 spot erosions |
| 4 | 1 or 2 linear erosions |
| 5 | Many small and a few large erosions |
| 6 | Extensive erosions over entire mucosa |

Determination of protective characteristics of the [Bi (OH)2] 8SOS against hydrochloric acid-induced gastric mucosal damage was undertaken. Rats were given 1 mL of the [Bi (OH)2] 8SOS orally fifteen minutes prior to oral administration of 1 mL of 0.6 M hydrochloric acid. One hour later, stomachs were removed, and the mucosal surface was examined macroscopically for damage. The degree of damage was again graded according to the scale presented in Table 1 above.

Determination of protective characteristics of the [Bi (OH)2] 8SOS in the Shay rat model was undertaken. Under ether anesthesia, the abdomen was opened, and the pylorus was ligated. Rats were immediately given 1 mL of the [Bi (OH)2] 8SOS; the abdomen was closed, and the animals were allowed to recover. Fourteen hours later, stomachs were removed, and forestomachs were examined macroscopically for damage. The degree of damage was graded according to the scale presented in Table 2 below.

TABLE 2

| GRADE OF OBSERVED FORESTOMACH DAMAGE | |
|---|---|
| Grade | Appearance of Forestomach |
| 0 | Normal |
| 1 | Redness |
| 2 | One or two "spot" lesions |
| 3 | One or two "deep" lesions |
| 4 | Many, many deep lesions |

The results of the ethanol-induced gastric mucosal damage study are presented in Table 3 below. Treatment with 95% ethanol caused severe mucosal damage as seen in the water pretreatment control group where the mean grade of gastric mucosal damage was 4.78 ± 0.26. The [Bi (OH)2] 8SOS pretreatment significantly reduced the severity of damage in this model to 3.10 ± 0.48.

TABLE 3

| PROTECTION AGAINST 95% ETHANOL-INDUCED GASTRIC MUCOSAL DAMAGE | | |
|---|---|---|
| Pretreatment | Grade | N |
| Water | 4.78 ± 0.26 | 9 |
| [Bi(OH)2]8SOS | 3.10 ± 0.48 (1) | 10 |

(1) Significantly lower than the water-treated group, $p < 0.05$

The results of the hydrochloric acid-induced gastric mucosal damage study are presented in Table 4 below. Treatment with 0.6 hydrochloric acid caused severe mucosal damage as seen in the water pretreatment control group where the mean grade of gastric mucosal damage was 4.70 ± 0.29. The [Bi (OH)2] 8SOS pretreatment significantly reduced the severity of damage in this model to 2.90 ± 0.12.

TABLE 4

| PROTECTION AGAINST 0.6 M HYDROCHLORIC ACID GASTRIC MUCOSAL DAMAGE | | |
|---|---|---|
| Pretreatment | Grade | N |
| Water | 4.70 ± 0.29 | 10 |
| [Bi(OH)2]8SOS | 2.90 ± 0.12 (1) | 10 |

(1) Significantly lower than the water-treated group, p<0.05

The results of the Shay rat study are presented in Table 5 below. Ligation of the pylorus caused severe damage to the forestomach in 14 hours as seen in the water- treatment control group where the mean grade of damage was 3.69 ± 0.17. Treatment with the [Bi (OH)2] 8SOS significantly reduced the severity of damage in this model to 2.15 ± 0.17. Note that seven of twenty (35%) of the rats in the water-treated control group died. The [Bi (OH)2] 8SOS treatment reduced mortality to 0% in this model.

TABLE 5

| PROTECTION IN THE SHAY RAT MODEL | | |
|---|---|---|
| Treatment | Grade | N |
| Water | 3.69 ± 0.17 | 13 (2) |
| [Bi(OH)2]8SOS | 2.15 ± 0.17 (1) | 15 |

(1) Significantly lower than the water-treated group, p<0.05
(2) 7 of 20 died.

Thus, the [Bi (OH)2] 8SOS proved to be effective in all three gastric mucosal damage models used in this study. Accordingly thus, the bismuth saccharides of this invention may be used in general for the treatment of peptic ulcers and/or gastritis in appropriate human subjects.


Example 3

The effectiveness of the [Bi (OH)2] 8SOS as from Example 1 as a Campylobacter-like organism growth inhibiting agent was determined by examining its actions in agar gel, proceeding by the Kirby-Bauer technique. Two isolates of Campylobacter pylori were obtained from human biopsy stomach tissue, and both were separately cultivated on agar gel samples which had the requisite well formed therein. Wells were filled with either a dilute aqueous ammonium hydroxide solution, which was a 10:1 mixture by volume of water and standard concentrated aqueous ammonium hydroxide (28 to 30 percent ammonia, or ca. 58 percent ammonium hydroxide or [Bi (OH)2] 8SOS dissolved in the dilute aqueous solution. The agar samples were checked for inhibition of growth of the Campylobacter pylori surrounding the wells. An inhibition zone value of 10 mm or more is considered to be significant inhibition. The following results were observed, table 6.

TABLE 6

| Agar well zone inhibition zones | | |
|---|---|---|
| STUDY | in Bi(OH)2]8SOS (Dil. NH4OH) | Dilute NH4OH |
| 1 | 16 mm | 0 mm |
| 2 | 18 mm, . 18 mm, 16 mm | 0 mm |

This demonstrates that the bismuth (phosph/sulf)ated saccharides are not only effective in inhibiting the growth of Campylobacter-like organisms, they are also generally capable of being dissolved in dilute aqueous ammonia solutions. This is surprising and unexpected, and it is highly significant because, as mentioned before, the immediate area surrounding a colony of these organisms in in vivo gastrointestinal mucosa is believed to have dilute aqueous ammonia present due to its production by the organisms. Morover, the bismuth (phosph/sulf)ated saccharides are unexpectedly capable of being pharmaceutically effective in such dilute aqueous ammoniacal environments against the Campylobacter-like organisms.

Example 4

An objective of this study was to determine if the [Bi (OH)2] 8SOS of Example 1 would amelioriate manifestations of Campylobacter pylori-associated gastritis in gnotobiotic piglets. The study was conducted in a generally blind format, and thus, the [Bi (OH)2] 8SOS is referred to in this example as "drug."

A litter of gnotobiotic piglets was derived by routine methods, birthed on Day 1, and was divided into 3 groups as follows: Group I (Piglet Nos. 1 - 5); Group II (Piglet Nos. 6 - 10); and Group III (Piglet No. 11). Each piglet weighed at least approximately 1 kg at birth. All piglets of Groups I and II were orally infectd with about one billion Colony Forming Unit of Campylobacter pylori broth culture on Day 7 after pre-treatment with cimetidine, which brought the stomach pH upward to reflect a more conducive environment for the introduction of the Campylobacter pylori. The piglet in Group III, the uninfected and untreated control, was housed in a separate isolation unit. (One billion = $10^9$)

Ten days after infection (Day 17), treatment was initiated for all piglets of Group I. For this, 2.0 g of drug was suspended in 7.10 mL of milk formula and placed in the feed pan. Thirty to forty minutes after consumption, the piglets were fed 150-200 mL of replacement milk formula. The infected, untreated piglets of Group II and the piglet of Group III received 7-10 mL of the otherwise same milk formula but without the drug. The treatment and the feedings were continued three times per day (7:30 a.m., 12:00 noon, 4:00 p.m ) for 10 days (days 18 through 27).

On Day 28, the stomach of each piglet was exteriorized, ligated at the esophagus and duodenum, transected and removed. Each stomach was opened longitudinally along the greater and lesser curvature. Gross lesions, e.g., the presence of lumenal mucus or lymphoid follicles were noted. Samples of gastric mucosa from four anatomical regions of the stomach (cardia, fundus, pylorus and antrum) were taken and streaked onto specialized agar plates for bacterial re-isolation. Adjacent full thickness samples of gastric tissue were then collected from each anatomic region, placed into individual labeled vials and fixed in 10 percent phosphate buffered formalin. Tissue samples were, respectively, trimmed, embedded in paraffin, sectioned at 5-6 u, and stained with hematoxylin and eosin and Warthin-Starry (W/S) stains for histologic evaluation and demonstration of any organisms.

Table 7 summarizes gross anatomic findings. i.e., a summary of gross observations in gastric tissue from the gnotobiotic piglets infected with Campylobacter pylori and treated with the drug. Changes from the normal, e.g. excess mucus and/or submucosal lymphoid follicles were detected in all 5 infected, untreated piglets (Group II) whereas, no lesions were detected in any of the five infected treated piglets (Group I) or in the uninfected, untreated control (Group III).

TABLE 7

| Gross Anatomic Findings | | |
|---|---|---|
| Identification | Excess Gastric Lumenal Mucus | Lymphoid Follicles |
| Group I: | | |
| Piglet No. 1 | 0 | 0 |
| Piglet No. 2 | 0 | 0 |
| Piglet No. 3 | 0 | 0 |
| Piglet No. 4 | 0 | 0 |
| Piglet No. 5 | 0 | 0 |
| Group II: | | |
| Piglet No. 6 | 1 | 3 |
| Piglet No. 7 | 1 | 0 |
| Piglet No. 8 | 0 | 2 |
| Piglet No. 9 | 0 | 2 |
| Piglet No. 10 | 0 | 1 |
| Group III: | | |
| Piglet No. 11 | 0 | 0 |

Table 7 is scored visually as: 0 indicates no lesions; 1 + indicates minimal change from normal; 2 + indicates moderate change from normal, and 3 + indicates severe change from normal.

Table 8 summarizes hisopathologic findings, i.e., a summary of histopathologic findings in gastric tissue from the gnotobiotic piglets infected with Campylobacter pylori and treated with the drug. The control piglet of Group III exhibited several small inactive lymphoid follicles below the muscularis mucosae, a condition found occasionally in such uninfected piglets. Inflammatory lesions associated with Campylobacter pylori-induced gastritis were composed of focal and diffuse lymphoplasmacytic cellular infiltrates in the lamina propria of the gastric mucosa. Cellular aggregates ranged from small focal collections of leukocytes to large, prominant well-organized lymphoid follicles. All infected piglets from both Groups I and II exhibited histologic evidence of gastritis. However, the severity and extent of the inflammation was substantially less in the drug-treated piglets of Group I when compared to those piglets not treated with the drug (Group II).

TABLE 8

| Histopathologic Findings | | | | |
|---|---|---|---|---|
| | Anatomical Region of the Stomach | | | |
| Identification | Cardia | Fundus | Antrum | Pylorus |
| Group I: | | | | |
| Piglet No. 1 | 2 | 0 | 2 | 0 |
| Piglet No. 2 | 2 | 0 | 1 | 0 |
| Piglet No. 3 | 1 | 1 | 1 | 0 |
| Piglet No. 4 | 3 | 1 | 0 | 0 |
| Piglet No. 5 | 0 | 0 | 2 | 0 |
| Group II: | | | | |
| Piglet No. 6 | 2 | 2 | 3 | 0 |
| Piglet No. 7 | 3 | 1 | 3 | 0 |
| Piglet No. 8 | 3 | 1 | 3 | 0 |
| Piglet No. 9 | 2 | 3 | 3 | 0 |
| Piglet No. 10 | 1 | 1 | 1 | 0 |
| Group III: | | | | |
| Piglet No. 11 | 0 | 0 | 0 | · 0 |

Table 8 is scored visually as: 0 indicates no microscopic lesions; 1 indicates minimal change from normal; 2 indicates moderate change from normal, and 3 indicates severe change from normal.

Table 9 summarizes findings related to the presence of the drug, i.e., a summary of the presence of the drug in the gastric lumenal mucus in the STET piglets infected with Campylobacter pylori and treated with the drug. In addition to inflammation, crystalline amorphous extracellular material indicating the presence of drug in the gastric mucus layer of all 5 treated piglets. This demonstrates that the drug was retained in the stomach tissue even on the day following cessation of treatment.

TABLE 9

| A summary of Findings Related to Presence of the Drug | | | | |
|---|---|---|---|---|
| | Anatomical Region of the Stomach | | | |
| Identification | Cardia | Fundus | Antrum | Pylorus |
| Group I: | | | | |
| Piglet No. 1 | + | + | + | + |
| Piglet No. 2 | - | - | + | + |
| Piglet No. 3 | - | - | - | + |
| Piglet No. 4 | + | + | + | + |
| Piglet No. 5 | + | + | + | + |
| Group II: | | | | |
| Piglet No. 6 | - | - | - | - |
| Piglet No. 7 | - | - | - | - |
| Piglet No. 8 | - | - | - | - |
| Piglet No. 9 | - | - | - | - |
| Piglet No. 10 | - | - | - | - |
| Group III: | | | | |
| Piglet No. 11 | - | - | - | - |
| The presence of amorphous, crystalline extracellular debris in gastric lumenal was detected histologically as " + " (present) or "-" (absent). | | | | |

Table 8 summarizes microbiologic findings, i.e., microbiologic findings in the gastric mucosaof the gnotobiotic piglets infected with Campylobacter pylori and treated with the drug. Organisms were recovered from all four anatomic sites in all five infected, untreated piglets and indentified as Campylobacter pylori on the basis of growth on TVAP, gram stain and urease and catalyase activity. Object organisms were not recovered from any treated piglet of Group I, nor were object organisms recovered from the control piglet of Group III. The Warthin-Starry stain delineates organisms in situ. The stain is capricious and interpretation is frequently complicated by silver grain precipitate. See, Blaser, supra., at page 378. Structures suggestive of the object organisms were observed in 4 of 5 treated piglets (Group I) and 5 of 5 untreated piglets (Group II). However, the object bacterial organisms were much more frequent in sections of mucose from the Group II piglets.

TABLE 10

Microbiologic Findings

Culture and Re-isolation (1)

| Identification | Anatomical Region of the Stomach | | | |
|---|---|---|---|---|
| | Cardia | Fundus | Antrum | Pylorus |
| Group I: | | | | |
| Piglet No. 1 | 0 | 0 | 0 | 0 |
| Piglet No. 2 | 0 | 0 | 0 | 0 |
| Piglet No. 3 | 0 | 0 | 0 | 0 |
| Piglet No. 4 | 0 | 0 | 0 | 0 |
| Piglet No. 5 | 0 | 0 | 0 | 0 |
| Group II: | | | | |
| Piglet No. 6 | 3 | 3 | 3 | 2 |
| Piglet No. 7 | 2 | 2 | 3 | 2 |
| Piglet No. 8 | 1 | 1 | 2 | 1 |
| Piglet No. 9 | 1 | 2 | 3 | 2 |
| Piglet No. 10 | 2 | 3 | 3 | 2 |
| Group III: | | | | |
| Piglet No. 11 | − | − | − | − |

(1) Data is expressed as a qualitative estimate of the number of colonies on TVAP agar as 0 indicating no growth, 1 indicating minimal growth, 2 indicating moderate growth, and 3 indicating extensive growth.

TABLE 10 (Continued)

Warthin-Starry (W/S) Stain (2)

Anatomical Region of the Stomach

| Identification | Cardia | Fundus | Antrum | Pylorus |
|---|---|---|---|---|
| Group I: | | | | |
| Piglet No. 1 | + | − | − | − |
| Piglet No. 2 | − | ± | − | − |
| Piglet No. 3 | + | ± | ± | − |
| Piglet No. 4 | − | − | − | − |
| Piglet No. 5 | − | + | + | − |
| Group II: | | | | |
| Piglet No. 6 | + | + | + | + |
| Piglet No. 7 | + | − | − | + |
| Piglet No. 8 | + | + | ± | + |
| Piglet No. 9 | ± | + | ± | + |
| Piglet No. 10 | + | + | + | + |
| Group III: | | | | |
| Piglet No. 11 | − | − | − | − |

(2) Data on W/S stain is scored as "+" indicating present, "−" indicating not present, or "±" indicating presence being questionable.

The following conclusions were drawn:
1. The drug was generally nontoxic and well-tolerated by the piglets.
2. All piglets of Groups I and II became infected with Campylobacter pylori.
3. Treatment with the drug in general effectively eliminated culturable Campylobacter pylori from gastric mucosa in all anatomic locations; residual organisms were detected rarely in W/S-stained sections. It was not known if these organisms were viable, or if they were organisms vs. stain precipitate.
4. Surprisingly, treatment appeared to result in diminution of histologic lesions associated with Campylobacter pylori gastric colonization.
5. The drug appears to be very effective in the treatment of Campylobacter pylori associated gastritis in this animal model system.
Thus, the [Bi (OH)2] 8SOS proved to be most significantly effective in the amelioration or control of Campylobacter pylori in the in vivo models used in this study. Accordingly, the bismuth (phosph/sulf)ated saccharides may be used in general as disclosed herein for analogous treatment in human subjects as well.

Conclusion

14

The present invention is thus provided. Various modifications can be effected by those skilled in the art within the spirit of this invention, the scope of which is particularly pointed out by the following distinctly claimed subject matter.

## Claims

1. A bismuth (phosph/sulf)ated saccharide.

2. A bismuth (phosph/sulf)ated saccharide according to claim 1, characterised in that the compound is a bismuth hydroxide (phosph/sulf)ated saccharide.

3. A bismuth (phosph/sulf)ated saccharide according to claim 2, characterised in that the compound is a bismuth hydroxide sulfated saccharide.

4. A bismuth (phosph/sulf)ated saccharide according to claim 3, characterised in that the saccharide moiety is a mono-, di-, tri- or tetra-saccharide.

5. A bismuth (phosph/sulf)ated saccharide according to claim 4, characterised in that the saccharide moiety is a di-saccharide which is polysulfated.

6. A bismuth (phosph/sulf)ated saccharide according to claim 4, characterised in that the saccharide moiety is substantially persulfated.

7. A bismuth (phosph/sulf)ated saccharide according to claims 5 or 6, characterised in that the saccharide moiety is sucrose.

8. A bismuth (phosph/sulf)ated saccharide according to claim 7, characterised in that the compound is bismuth hydroxide sucrose octasulfate.

9. A pharmaceutical composition characterised in that it contains a bismuth (phosph/sulf)ated saccharide and a pharmaceutically acceptable carrier.

10. The use of a bismuth (phosph/sulf)ated saccharide for the manufacture of a medicament for the amelioration of disorders associated with gastric mucosal damage.

11. The use of a bismuth (phosph/sulf)ated sacchride for the manufacture of a medicament for the amelioration of gastointestinal disorders associated with a Campylobacter-like organism.

12. A pharmaceutical composition according to Claims 9, 11 or 12 characterised in that the bismuth (phosph/sulf) ated saccharide is according to any of the claims 1 to 8.

13. A pharmaceutical composition according to claims 9, 11 or 12 characterised in that it is in oral dosage form.

14. A method of making a bismuth (phosph/sulf)ated saccharide characterised in contacting a hydrogen (phosph/sulf)ated saccharide and a bismuth substance under conditons such that the bismuth (phosph/sulf)ated saccharide is prepared.